# EUROPEAN PATENT SPECIFICATION

(11) **EP 0 512 066 B1**
(45) Date of publication and mention of the grant of the patent: **13.11.1996**
(21) Application number: 91904443.8
(22) Date of filing: 22.01.1991
(51) Int. Cl.: C12N 5/00, C12N 5/02, C12Q 1/02

(54) **CO-INDEPENDENT GROWTH MEDIUM FOR MAINTENANCE AND PROPAGATION OF CELLS**
CO-UNABHÄNGIGES NÄHRMEDIUM ZUR HALTUNG UND VERMEHRUNG VON ZELLEN
MILIEU DE CULTURE CO-INDEPENDANTE POUR CONSERVER ET MULTIPLIER DES CELLULES

(30) Priority: 22.01.1990 US 467939
(43) Date of publication of application: 11.11.1992
(73) Proprietor: THE UNITED STATES OF AMERICA as represented by the Secretary UNITED STATES DEPARTMENT OF COMMERCE, Washington, DC 20231 (US)
(72) Inventor: VISTICA, David, Thomas, Monrovia, MD 21770 (US); SKEHAN, Philip, James, Boonsboro, MD 21713 (US); SCUDIERO, Dominic, Albert, Frederick, MD 21780 (US); BOYD, Michael, Ray, Ijamsville, MD 21754 (US); MONKS, Anne, Patricia, Clarksburg, MD 21713 (US)
(74) Representative: Jump, Timothy John Simon
(86) International application number: US9100451
(87) International publication number: WO9110726

(56) References cited:
- US-A- 3 883 393
- US-A- 4 411 990
- US-A- 4 533 637
- PROCEEDINGS OF THE EIGHTIETH ANNUAL MEETING OF THE AMERICAN ASSOCIATION FOR CANCER RESEARCH vol. 30, March 1989, page 613 VISTICA D.T. ET AL 'Development and evaluation of a CO2-independant culture medium for use in a high flux in vitro anticancer drug screen employing a broad panel of human tumor cell lines'
- JOURNAL OF CELLULAR PHYSIOLOGY vol. 100, no. 3, 1979, NEW YORK pages 548 - 550 C. WAYMOUTH 'Autoclavable medium AM 77B'
- R. IAN FRESHNEY 'Culture of animal cells. A manual of basic technique' 1987 , ALAN R. LISS, INC. , NEW YORK
- Science, Volume 224, issued 29 June 1984, "Human Colon Cells: Culture and in vitro Transformation", pages 1445-1447
- "GIBOO Laboratories Catalogue and Reference Guide", Published 1987 by GIBOO Laboratories, Life Technologies, Inc. (N.Y.), see page 147, Liebovitz' L-15 Media
- Fudamentals of Analytical Chemistry, 4th edition, D.A.Skoog, 1982, pages 39-48
- Gene Print TM, pages 40-44
- Bathesda Research Laboratories Catalogue & Reference Guide, 1988, page 77
- Gene Print DNA Typing Technical Bulletin, pages 1-8

## Description

The present invention is related generally to cellular growth media. More particularly, the present invention is related to providing a growth medium which allows maintenance and propagation of cells in normal atmospheric CO₂, that is without requiring- an exogenous, regulated supply of enriched carbon dioxide for buffering.

Conventional growth media, containing bicarbonate ion as the primary buffering component, require a special carbon dioxide atmosphere (to maintain physiological pH of about 7.2-7.4). This necessitates expensive incubators with a controlled atmosphere and has the disadvantage that normal working manipulations, which must be done outside of this special atmosphere, lead to rapid changes in the pH of the medium which, in turn, stress cells and alter their functionality.

Several culture media for propagation of cells in atmospheric CO₂ are commercially available. Of these, RPMI 1630 was found to be unsatisfactory in the current study for propagation of cell lines representing 7 tumor histologies. L-15 appears to be superior to RPMI 1630 for culture of tumor cells in atmospheric CO₂ without added bicarbonate and a CO₂ enriched atmosphere. However, L-15 medium is inadequate for growing many kinds of human tumor cells and was found to be incompatible with drug toxicity evaluation utilizing tetrazolium reduction endpoints due to the lack of the sugar D-glucose in its formulation.

In abstract No. 2440 of the Proceedings of the Eightieth Annual Meeting of the American Association for Cancer Research, Vol. 30, 03-1989, page 613, a culture medium for culturing tumor cells under atmospheric CO₂ is disclosed. This culture medium is said to derive its buffering capacity from β-glycerophosphate and to be optimized to facilitate growth of cells in atmospheric CO₂ by inclusion of biotin, L-asparagine, pyruvate oxaloacetate. However, no further details of the medium's compositions are given.

It is, therefore, an object of the present invention to provide a cellular growth medium which, unlike heretofore known growth media, supports the growth of a wide variety of tumor and non-tumor cells of human and non-human origin in a non-CO₂ enriched atmosphere.

It is a further object of the present invention to provide a CO₂-independent culture medium suitable for in vitro anticancer drug screen, employing a broad panel cf human tumor cell lines.

Another object of the present invention is to provide a CO₂-independent culture medium suitable for a wide variety of cell culture technologies where employing CO₂ to control the medium pH is cumbersome, expensive, impractical or undesirable.

Various other objects and advantages will become evident from the following detailed description of the invention.

These and other objects, features and many of the attendant advantages of the invention will be better understood upon a reading of the following detailed description when considered in connection with the accompanying drawings wherein:

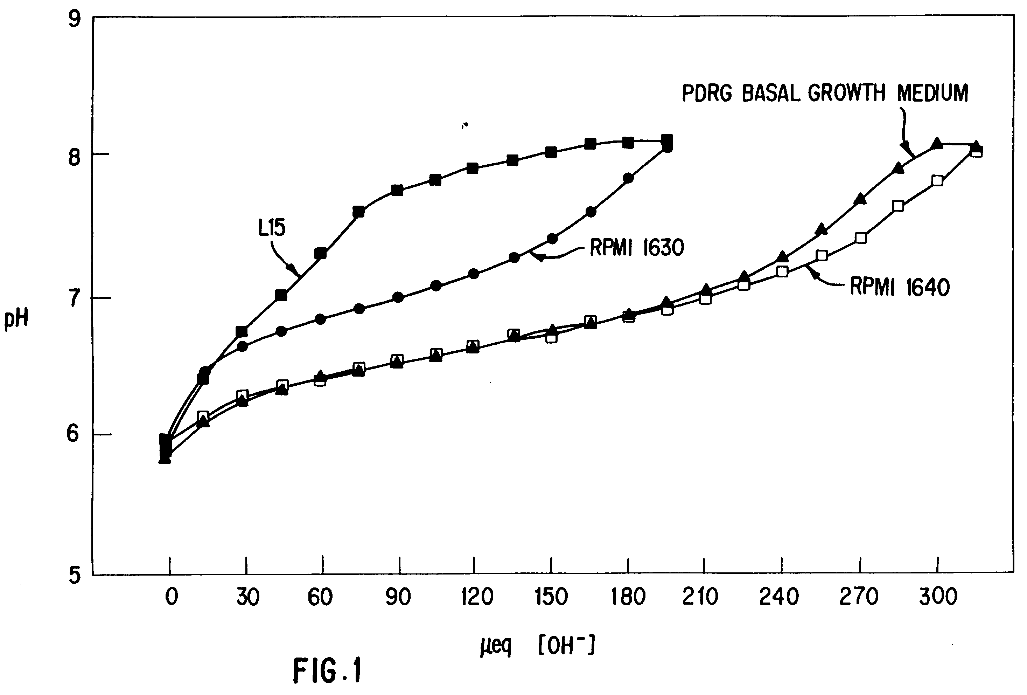

Figure 1 shows the comparative results of the buffering capacity of RPMI 1640, RPMI 1630, L-15, and PDRG basal growth medium. Fifteen ml of serum-free RPMI 1640 (□), RPMI 1630 (●), L-15 (■), and PDRG basal growth medium (▲) was adjusted to pH 6.0 with 1 N HCl. Sodium hydroxide (1 N) was added in 15 µl aliquots and the pH determined.

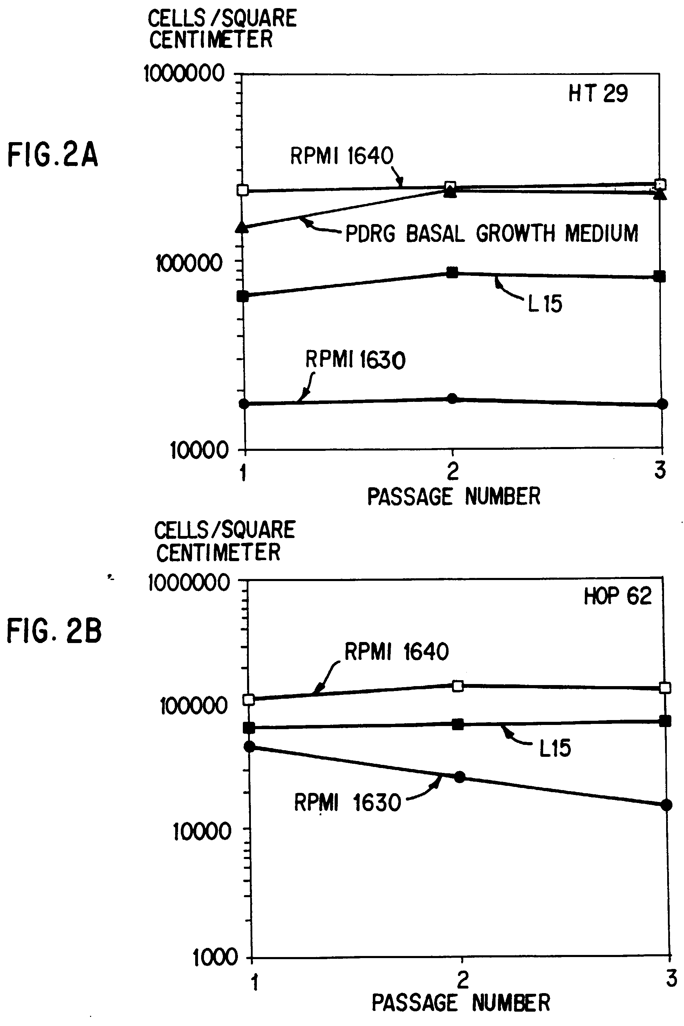

Figures 2A-2G show the comparative results of the adaption of Human Tumor Cell Lines to Growth in Atmospheric CO₂. Cell lines, in RPMI 1640 growth medium, were trypsinized and subcultured weekly in RPMI 1640 medium (□) for growth in 5% CO₂ or in RPMI 1630 (●), L-15 (■) or PDRG basal growth medium (▲) for growth in atmospheric CO₂.

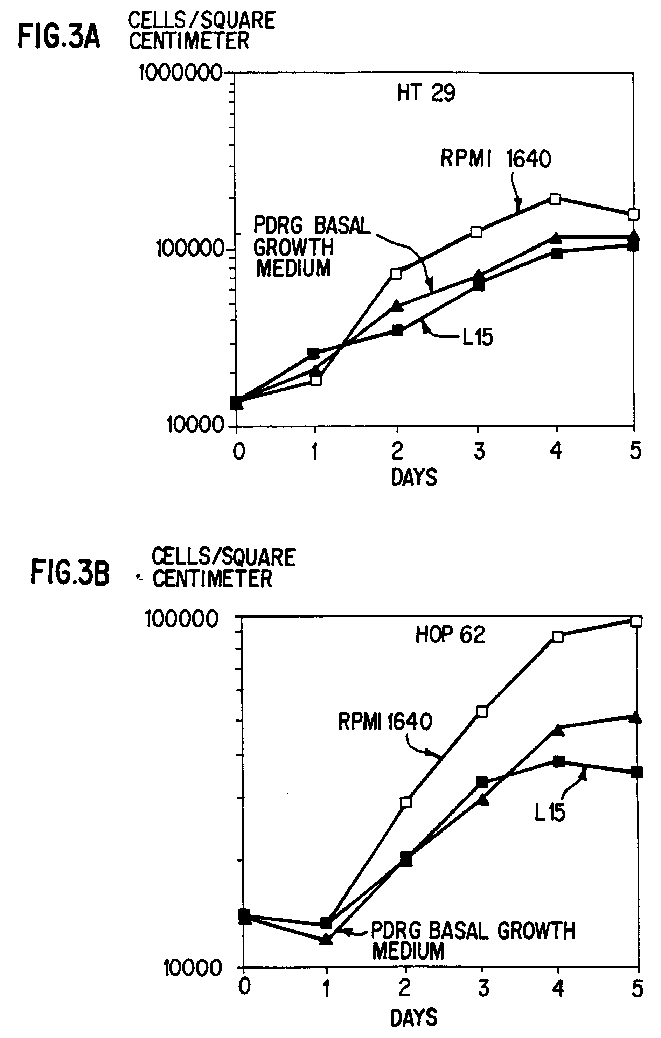

Figures 3A-3G show the comparative growth curves of Human Tumor Cell Lines in Atmospheric CO₂. Cell lines, in RPMI 1640 medium (□), L-15 medium (■), or PDRG basal growth medium (▲), were trypsinized, seeded into 25 cm² flasks and counted at 24 hour intervals.

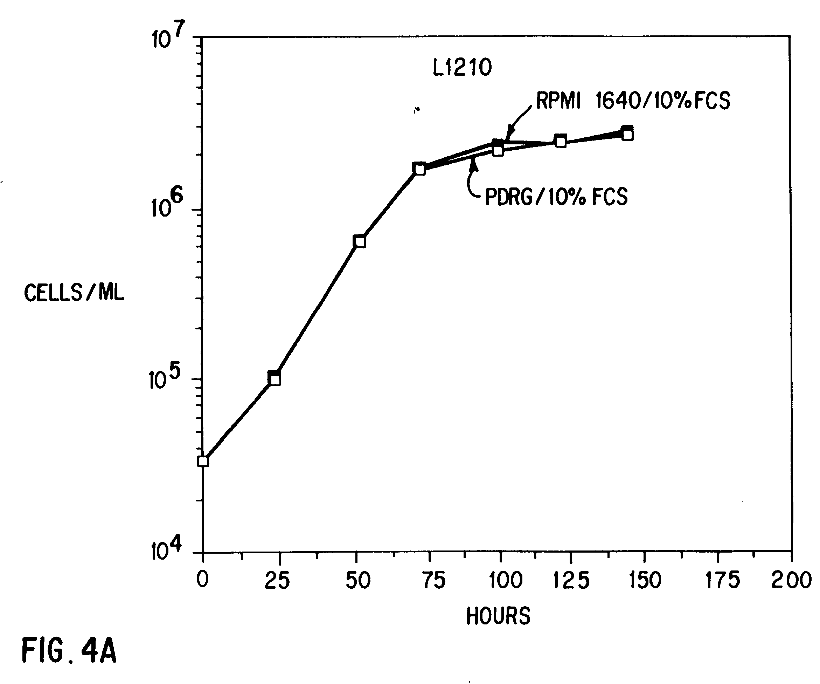

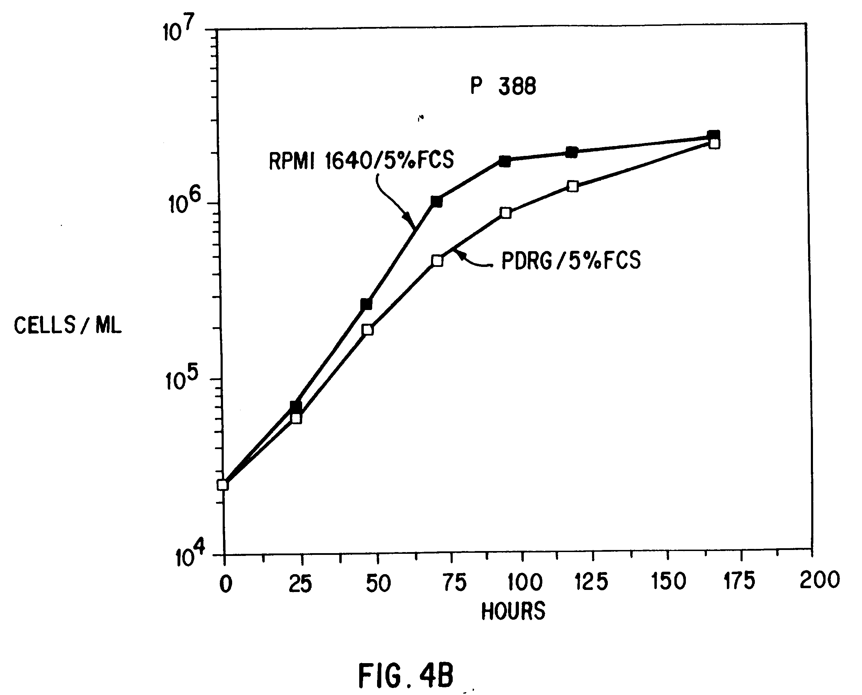

Figures 4A and 4B show the growth of the P388 and L1210 murine lymphocytic leukemias in atmospheric CO₂ in PDRG basal growth medium (□) or in RPMI 1640 medium (■) in 5% CO₂.

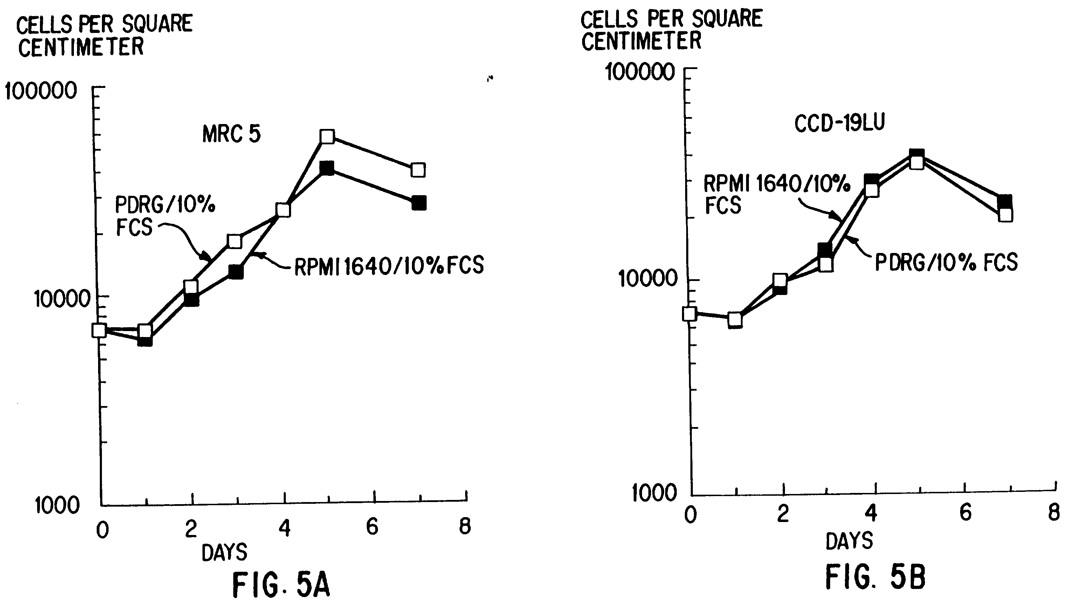

Figures 5A-5E show the growth of 4 human fibroblast cell lines (MRC-5, CCD-19 LU, MAR BEL and IMR-90) and one murine fibroblast (3T3) in PDRG basal growth medium (□) in atmospheric CO₂ or in either RPMI 1640 (■) medium (human fibroblasts) or Dulbecco's Modified Eagles Medium (DMEM) (3T3 murine fibroblast in 5% carbon dioxide.

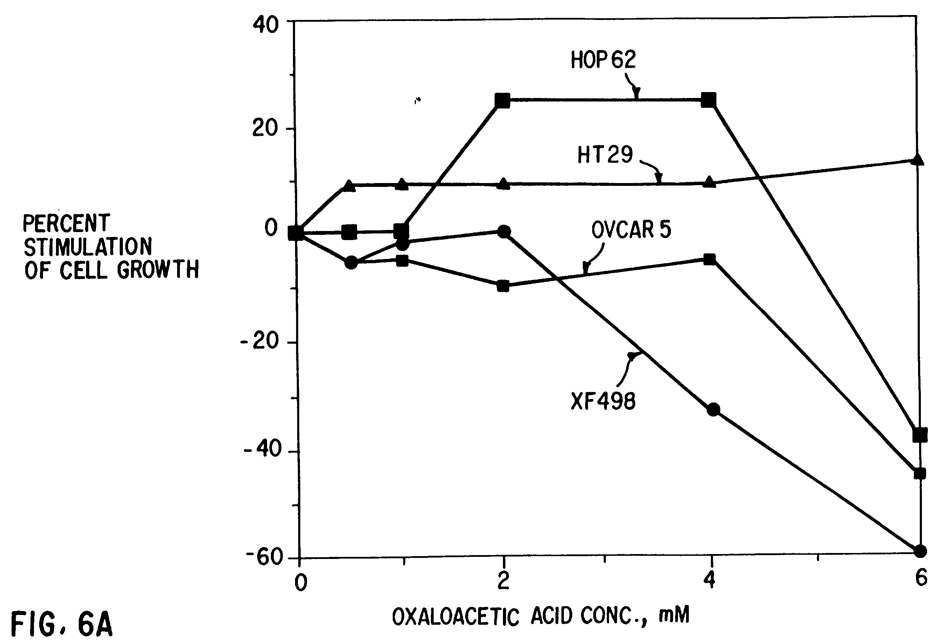

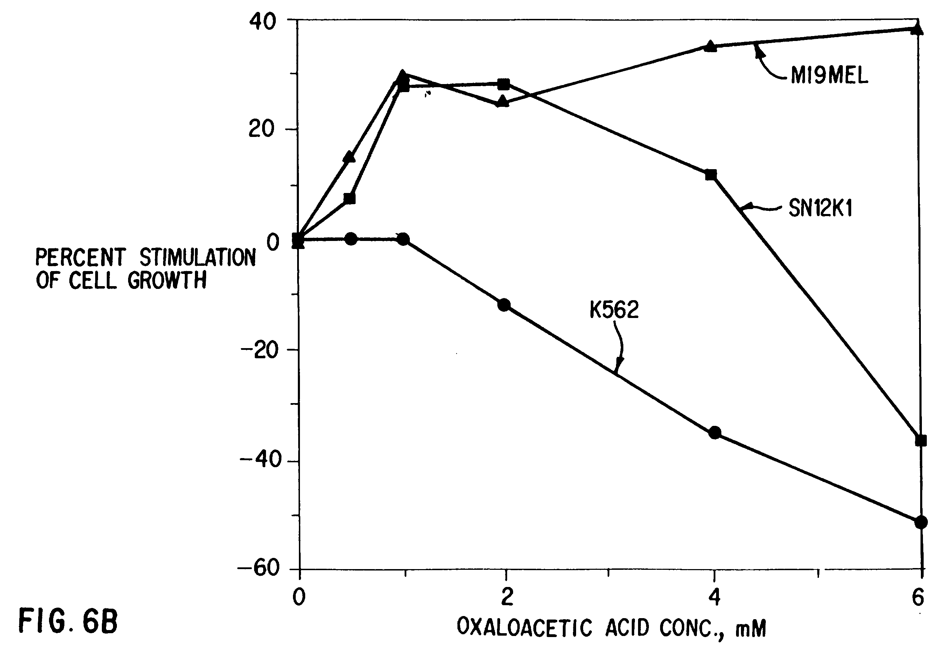

Figures 6A and 6B show the stimulation of Cellular Proliferation in Atmospheric CO₂ by Oxaloacetic Acid. Cell lines, in PDRG basal growth medium, were exposed to the indicated concentration of oxaloacetic acid. Cells were trypsinized and counted five days later.

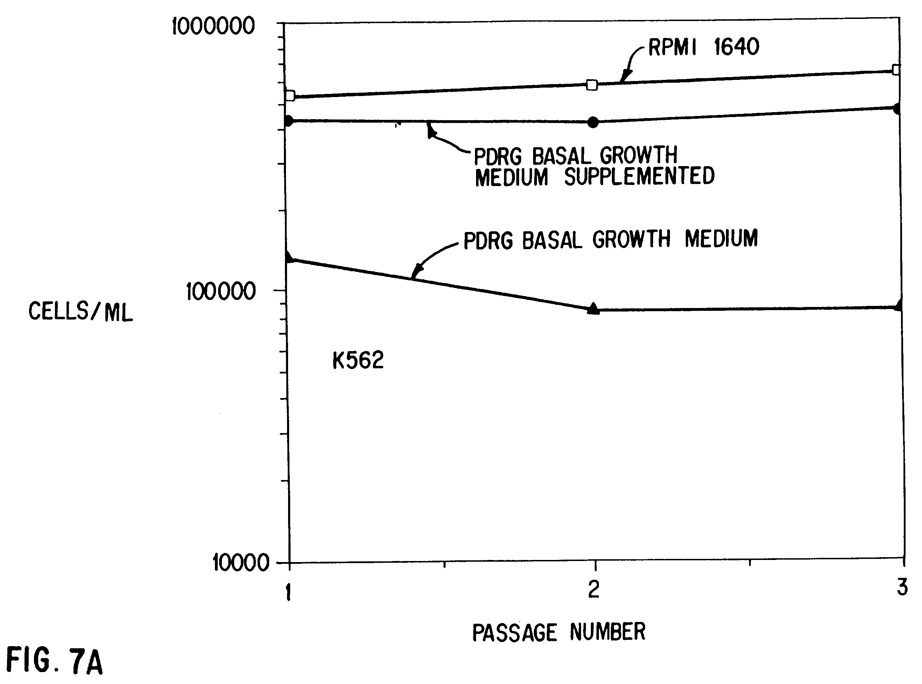

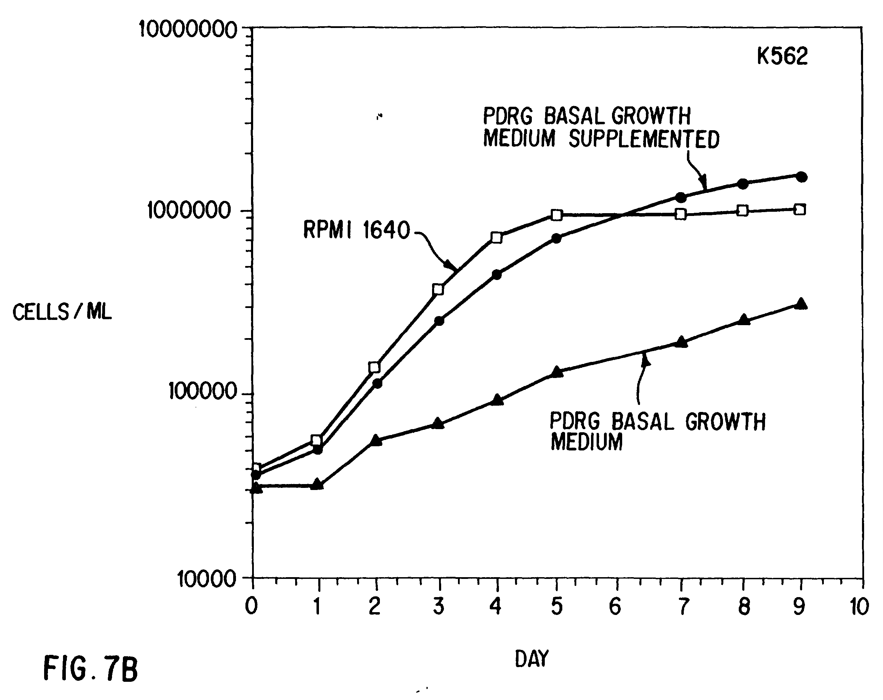

Figures 7A and 7B show the optimization of PDRG basal growth medium for Propagation of Leukemia Cells in Atmospheric CO₂. Top Panel: K562 chronic myelogenous leukemia cells, in RPMI 1640 growth medium, were passaged weekly in RPMI 1640 medium (□), PDRG basal growth medium (▲) or in PDRG basal growth medium supplemented (●) with, in mg/L, adenosine (10), Cytidine (10), Guanosine (10), Uridine (10), Inosine (25), and Orotic Acid (15). Bottom Panel: Cells were harvested following 3 passages in the respective medium, seeded at the indicated concentration in the same medium, and harvested for daily cell counts.

The above and various other objects and advantages of the present invention are achieved by the formulation of a basal medium for growth or maintenance of cells in atmospheric CO₂ and composed essentially of the ingredients listed in Table 2. The basal medium of the present invention is designated herein as PDRG basal growth medium.

Accordingly, in a first aspect of the invention there is provided a growth medium for the maintenance, growth or propagation of cells in a CO₂-independent atmosphere, composed of the following ingredients:

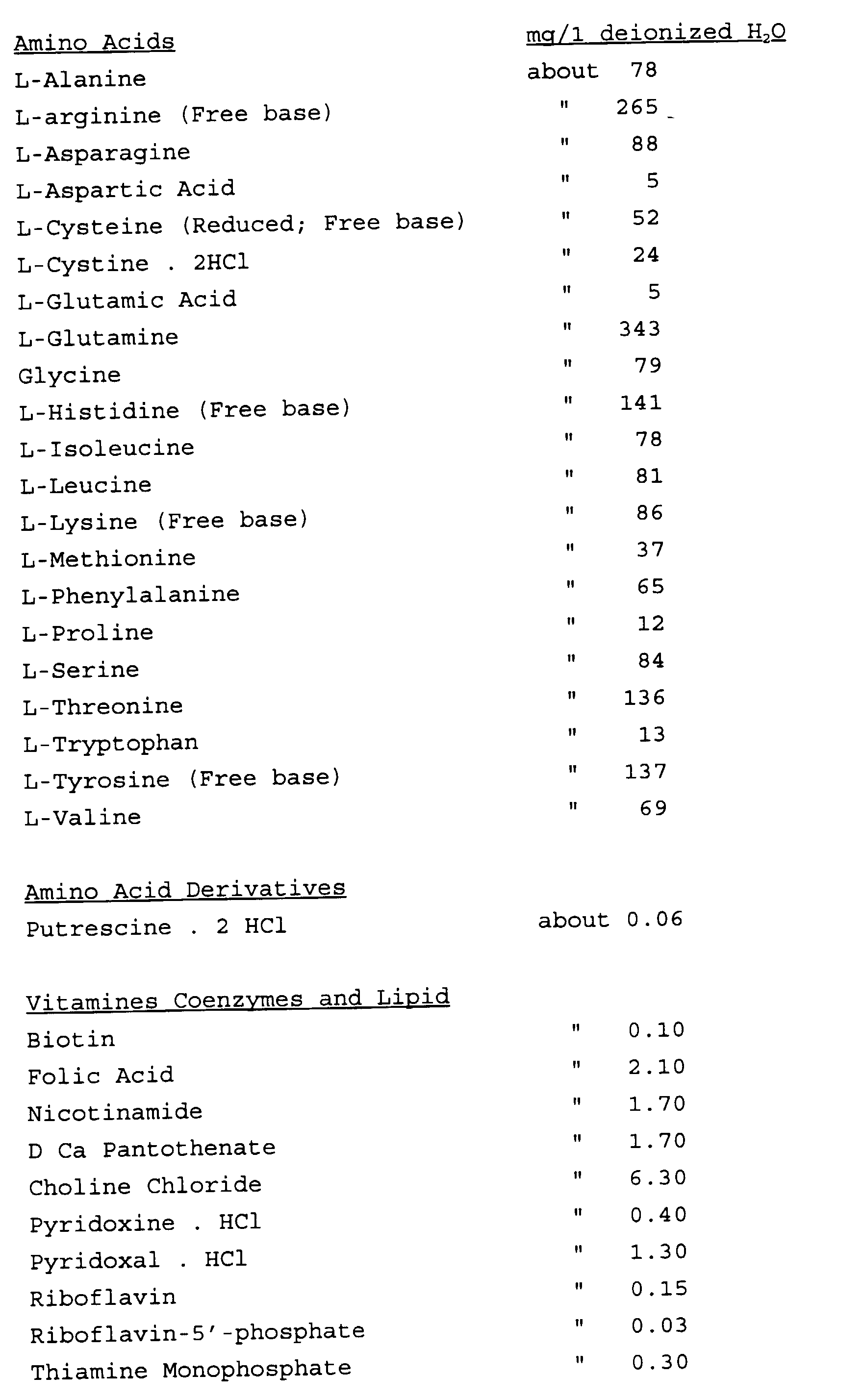

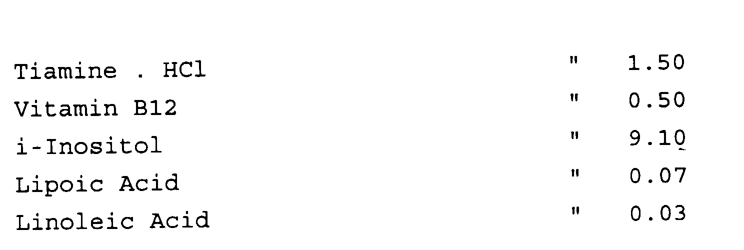

| Carbohydrates and Derivatives | mg/l deionized water |
|---|---|
| D-Glucose | about 1000 |
| Sodium Pyruvate | about 257 |

| Nucleic Acid Derivatives | |
|---|---|
| hypoxanthine | about 1.4 |
| Thymidine | about 0.24 |

| Inorganics | |
|---|---|
| NaCl | about 7000 |
| KCl | about 341 |
| Na₂ HPO₄ (ANHY) | about 111 |
| NaH₂PO₄ (ANHY) | about 36 |
| MgSO₄ (ANHY) | about 66 |
| MgCl₂ . 6H₂O | about 107 |
| KH₂PO₄ | about 20 |
| CaCl₂ . 2H₂O | about 165 |

| Inorganic Trace Elements | |
|---|---|
| CuSO₄ . 5H₂O | about 0.0008 |
| Fe(NO₃)₃ . 9H₂O | about 0.03 |
| FeSO₄ . 7H₂O | about 0.28 |
| ZnSO₄ . 7H₂O | about 0.29 |

| Buffers and Indicators | |
|---|---|
| NaHCO₃ | not exceeding 75 |
| β-glycerophosphate | about 4320 |
| Phenol Red | about 5. |

and having a final pH of about 7.3 and final osmolarity of about 290-295 mos/Kg.

In a second aspect of the invention, there is provided a method for culturing cells, comprising growing said cells in a growth medium according to the invention in its first aspect in a CO₂-independent atmosphere.

In a third aspect of the invention, there is provided a method for in vitro screening of anticancer agents, comprising growing tumor cells in vitro in a growth medium according to the invention in its first aspect in the absence and presence of an anticancer agent, wherein a decrease in growth of the cells in the presence of the anticancer agent, is indicative of the anticancer potency of said agent.

Preferred embodiments of the invention in any of its various aspects are defined in the sub-claims.

Unless defined otherwise, all technical and scientific terms used herein have the same meaning as commonly understood by one of ordinary skill in the art to which this invention belongs. Unless mentioned otherwise, the techniques employed or contemplated herein are standard methodologies well known to one of ordinary skill in the art.

The term "atmospheric CO₂" as used herein means the CO₂ content that naturally occurs in the atmospheric air at normal temperature and pressure.

The term "CO₂-independent" as used herein means that no exogenous, regulated supply of enriched CO₂ is required.

The term "cells" as used herein means human or non-human, tumor or non-tumor cells required to be grown, maintained or propagated in an in vitro system in a CO₂-independent atmosphere.

Unless indicated otherwise, all materials were easily obtained from commercial sources.

### Comparative Buffering Capacity of RPMI 1640, RPMI 1630, L-15 and PDRG Basal Growth Medium

Fifteen ml of serum-free medium was adjusted to pH 6.0 with 1 N HCl. Sodium hydroxide (1 N) was added in 15 µl aliquots and the pH determined following vigorous vortexing.

### Adaption of Cell Lines to Growth in Atmospheric CO₂

Cell lines (Table 1) were obtained from the tumor repository, (Frederick, MD). Human tumor cell lines were routinely grown in RPMI 1640 medium (Quality Biological, Gaithersburg, MD) containing 5% fetal bovine serum (Hyclone Laboratories, Logan, UT). Cultures were maintained in 75 cm² Costar culture flasks in a humidified atmosphere of 95% air, 5% carbon dioxide and were passaged at 85-90% of confluency. Cells were removed by trypsinization and subcultured according to following procedure: Medium was decanted and the cells rinsed once with 2 ml 0.05% trypsin containing 0.1% EDTA (Quality Biological, Gaithersburg, MD). The cells were then overlayed for 2-5 min. with 1.5 ml of a trypsin solution which was prepared by adding 1 ml of a 1% trypsin solution (Sigma Type III, 2X recrystallized) in a Ca²⁺/Mg²⁺ free phosphate buffered saline to 50 ml 0.05% trypsin containing 0.05% EDTA. Tumor cells were plated in 75cm² Costar culture flasks at a concentration of 1 x 10⁴ cells/cm² in 30 ml RPMI 1640, RPMI 1630 (Quality Biological, Gaithersburg, MD), L-15 (Quality Biological, Gaithersburg, MD) or PDRG basal growth medium containing 5% fetal bovine serum. Cells were maintained in a humidified atmosphere at 37°C in 5% CO₂ (RPMI 1640) or atmospheric CO₂ (RPMI 1630, L-15 and PDRG basal growth medium). Cells were trypsinized and subcultured weekly in the respective media for a period of four weeks.

All cell counts were performed on a Model ZM Coulter Counter (Hialeah, FL).

Four human fibroblast cell lines (MRC-5, CCD19-LU, Mar Bel and IMR-90) and 1 murine fibroblast (3T3) were adapted to growth in atmospheric CO₂ in PDRG basal growth medium containing 10% fetal calf serum. Growth curves of each cell line in 5% CO₂ in RPMI 1640 medium containing 10% fetal bovine serum and in PDRG basal growth medium in atmospheric CO₂ were performed as indicated below. The L1210 and P388 murine lymphocytic leukemia were similarly adapted to growth in PDRG basal growth medium containing 10% fetal bovine serum.

### Growth Curves of Cell Lines in Atmospheric CO₂

Growth curves of cell lines in L-15 and PDRG basal growth medium were performed following adaption of cells to growth in normal atmospheric CO₂ in the respective medium for a period of six weeks. Cells were trypsinized as described above and plated at a density of 7 x 10⁵ cells/cm² in 25 cm² Costar tissue culture flasks containing 7 ml of medium. Cells were trypsinized at 24 hour intervals and counted as described above.

### Promotion of Cellular Proliferation in Atmospheric CO₂ by Oxaloacetic Acid

The effect of oxaloacetic acid upon proliferation of cell lines was examined in the following manner. Cells utilized for these experiments had been adapted to growth in atmospheric CO₂ in PDRG basal growth medium. Cells were trypsinized, seeded into 25 cm² Costar flasks containing 7 ml PDRG Basal growth medium at a concentration of 1.4 x 10⁴ cells/cm². Twenty-four hours later, oxaloacetic acid was added to a final concentration of 0.1 mM to 6.0 mM. Oxaloacetic acid (Sigma Chemical Co., St. Louis, MO) was prepared as a 200 mM stock solution in distilled water, the pH adjusted to 6.5 with NaOH and the resulting stock solution stored at -80°C until use.

### Microculture Tetrazolium and Sulforhodamine B (SRB) Assays for Quantitation of In Vitro Drug Effects in Atmospheric CO₂

Tests were done to determine whether dose response curves of selected drugs performed with cells grown in an exposed to drugs in 5% CO₂ environment and were comparable to those obtained with cells grown in and exposed to the respective drug in atmospheric CO₂. Adriamycin, BCNU and tamoxifen were obtained from the Drug Prep Laboratory at the National Cancer Institute, Frederick Cancer Research Facility. Dose response curves were performed with cells maintained in RPMI 1640 medium under 5% CO₂ and in PDRG basal growth medium in atmospheric CO₂. Drug effects were quantitated following a 3 day drug exposure by both the MTT tetrazolium (Alley et al, 1988, Cancer Res., 48:589-601) and the SRB protein assay (Skehan et al, 1989, Proc. Amer. Assoc. Cancer Res., 30:612). Briefly, cold 50% trichloroacetic acid was gently layered on top of growth medium to a final concentration of 10% and the cells were fixed for 60 minutes at 4°C. Fixed cells were rinsed five times with tap water and stained for 15 minutes with 0.4% SRB (Sigma Chemical Col, St. Louis, MO) in 1% acetic acid. Unbound dye was removed by rinsing cells five times with 1% acetic acid and the stained cells were air dried. The bound dye was extracted with 10 mM unbuffered Tris base (pH 10 to 10.5) and the absorbance was read at 564 nM.

### Medium Formulation: Selection of the Primary Buffers and Optimization for Cellular Growth in Atmospheric CO₂

The HT 29 colon adenocarcinoma was utilized as a representative cell line to test the formulation of PDRG basal growth medium (Table 2) in atmospheric CO₂. The present formulation incorporates high concentrations of the free bases of several L-amino acids: arginine, cysteine, histidine, lysine and tyrosine. In addition to utilizing high concentrations of these amino acids, PDRG basal growth medium was buffered by addition of 20 mM β-glycerophosphate. PDRG basal growth medium was optimized for growth of cells in atmospheric CO₂ by incorporation of biotin, low concentrations of bicarbonate ion, pyruvic acid, asparagine and oxaloacetic acid into the medium. The requirement of bicarbonate ion for several biochemical processes including fatty acid biosynthesis necessitated its inclusion in the formulation of PDRG basal growth medium at a concentration (75 mg/L) in excess of that provided by serum. This was the maximum concentration of bicarbonate which could be added to the medium which did not require exogenous CO₂ buffering to maintain stable pH.

### Comparative Buffering Capacity of RPMI 1640, RPMI 1630, L-15 and PDRG Basal Growth Medium

The base titration of an acidified aliquot (pH 6.0) of each culture medium provided a measure of relative buffering capacity (Figure 1). PDRG basal growth medium was identical in this regard, to RPMI 1640, the bicarbonate based culture medium, and its resistance to alkalinization was substantially greater than either RPMI 1630 or L-15. Base titration of RPMI 1630 and L-15 indicated that the equivalents of [OH-] required to increase the pH of the medium from 6.0 to 7.5 was 1.6 and 3.7-fold less, respectively, than that observed with PDRG basal growth medium.

### Adaption of Human Tumor Cell Lines to Growth in Atmospheric CO₂

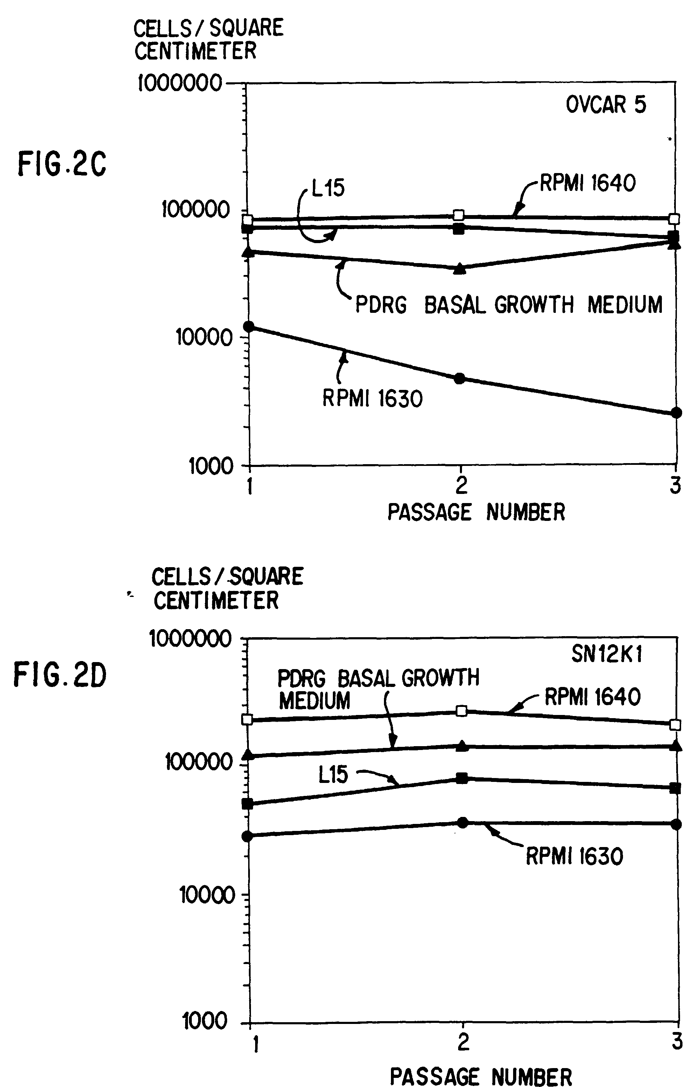

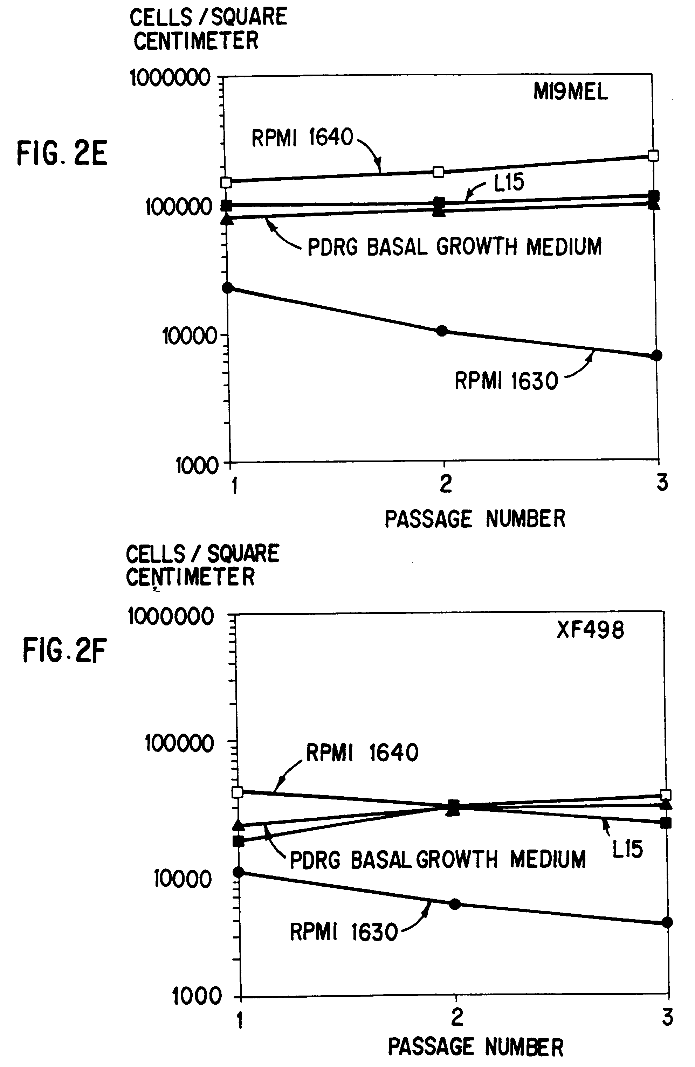

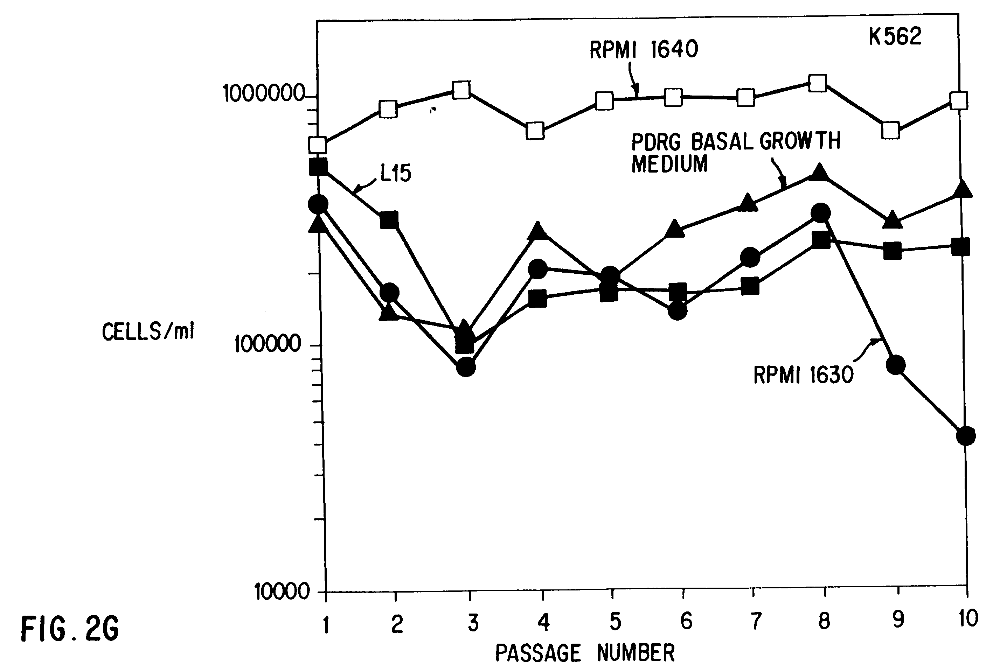

The results of tests designed to examine the feasibility of propagating human tumor cell lines, representing a broad spectrum of disease types, in atmospheric CO₂ are presented in Figure 2. Initial experiments were undertaken with the HT 29 colon adenocarcinoma. Growth profiles in RPMI 1630, L-15 and PDRG basal growth medium in atmospheric CO₂ were compared to those obtained with cells grown in RPMI 1640 medium under 5% CO₂. These results indicated that removal of HT 29 from a 5% CO₂ environment and growth in atmospheric CO₂ in PDRG basal growth medium was initially accompanied by a 50% reduction in cell growth. This was in contrast to 3.7- and 125-fold reductions in growth of HT 29 cells cultured in L-15 and RPMI 1630 media, respectively. Stable growth was obtained following 3-4 passages in the respective medium and resulted in nearly equivalent growth of the HT-29 cells in PDRG medium in atmospheric CO₂ and RPMI 1640 medium in 5% CO₂. The results of similar adaption experiments with other panel cell lines (Figure 2) indicate that (2) RPMI 1630 medium was unsatisfactory for propagation of these cell lines in atmospheric CO₂; (b) densities of cells grown in 5% CO₂ were higher than those obtained for cells grown in atmospheric CO₂; and (c) PDRG basal growth medium was superior to L-15 medium for growth of the K562 myelogenous leukemia, the HT 29 colon adenocarcinoma and the SN12K1 renal carcinoma. Cell densities were equivalent in PDRG basal growth medium and L-15 medium for the HOP 62 lung adenocarcinoma, the XF 298 glioblastoma and the M19Mel Melanoma. L-15 medium appeared to be superior to PDRG basal growth medium only for OVCAR-5, an ovarian carcinoma cell line.

### Growth Curves of Cell Lines in Atmospheric CO₂

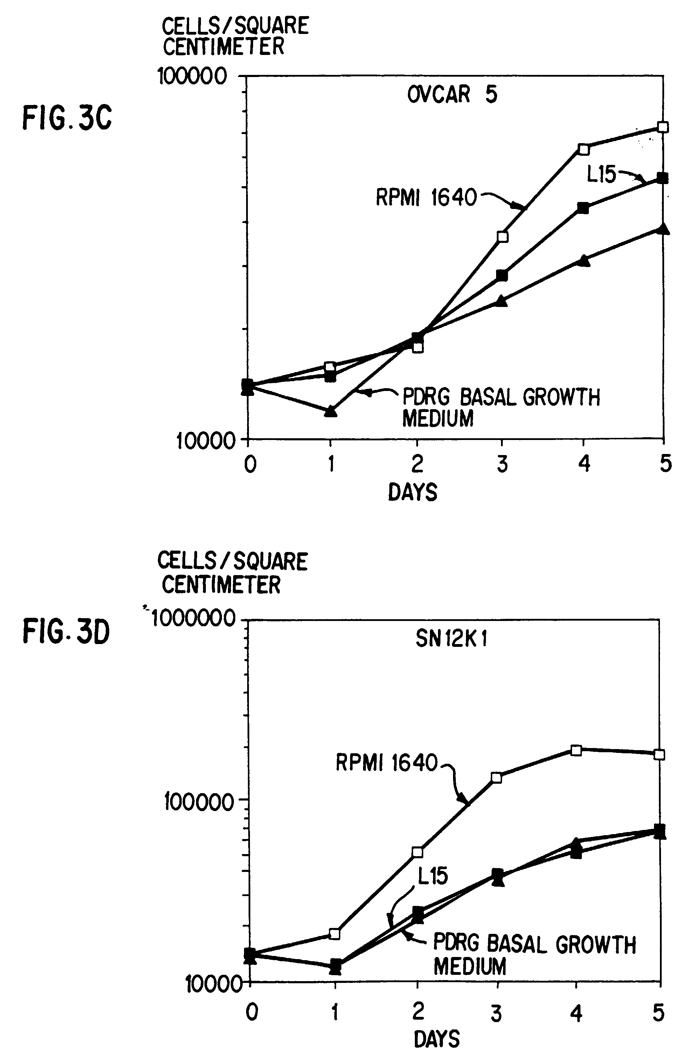

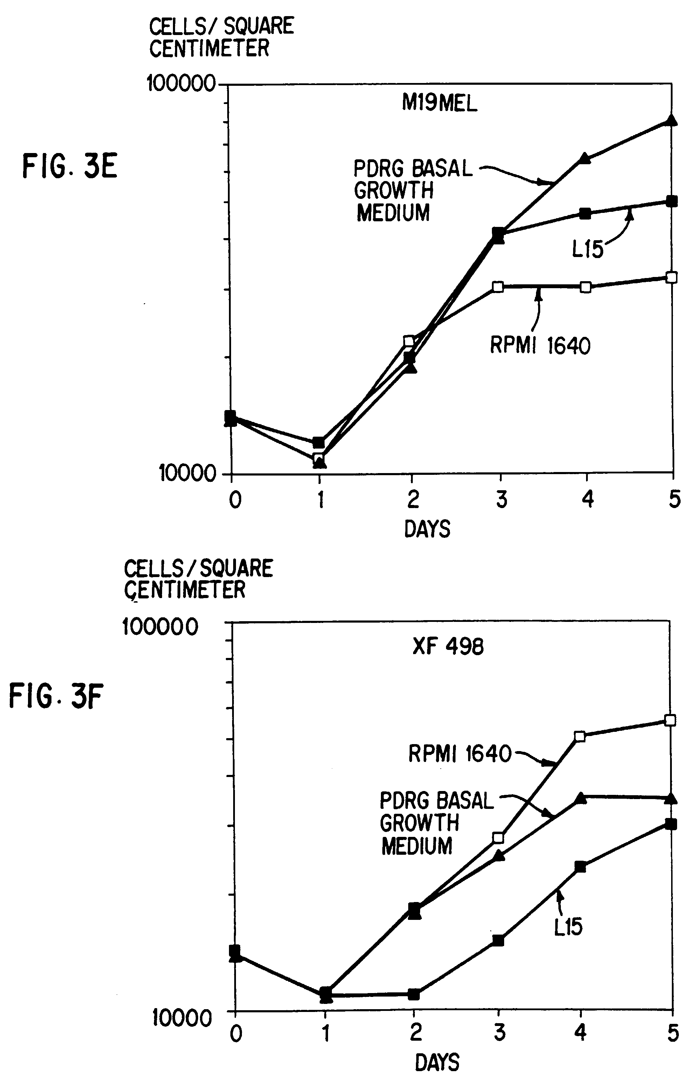

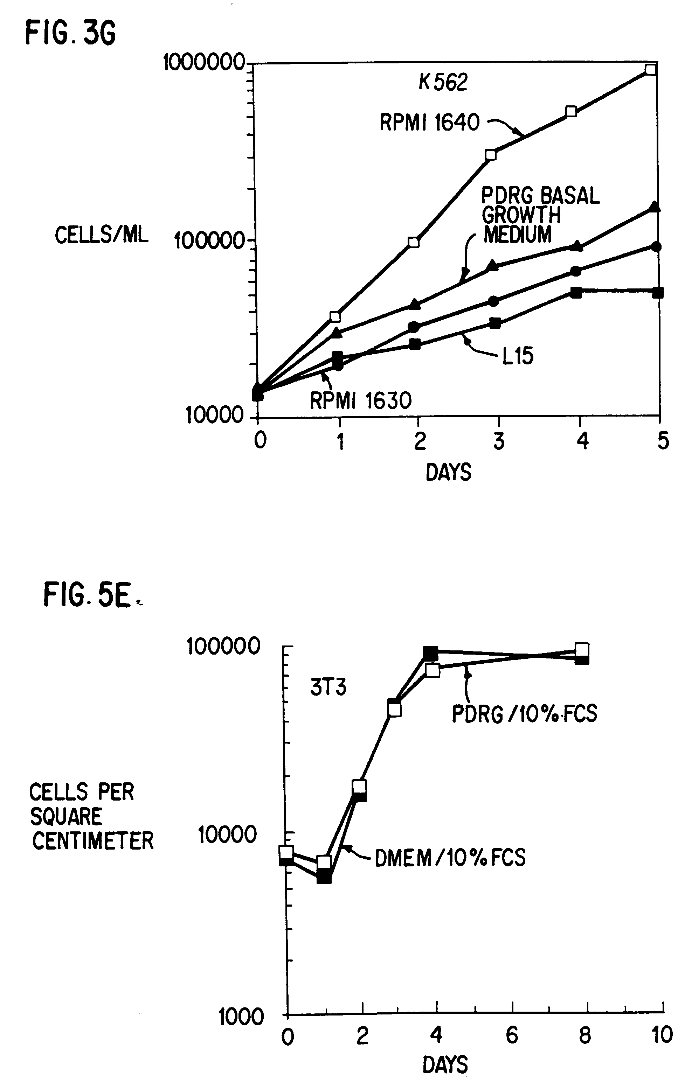

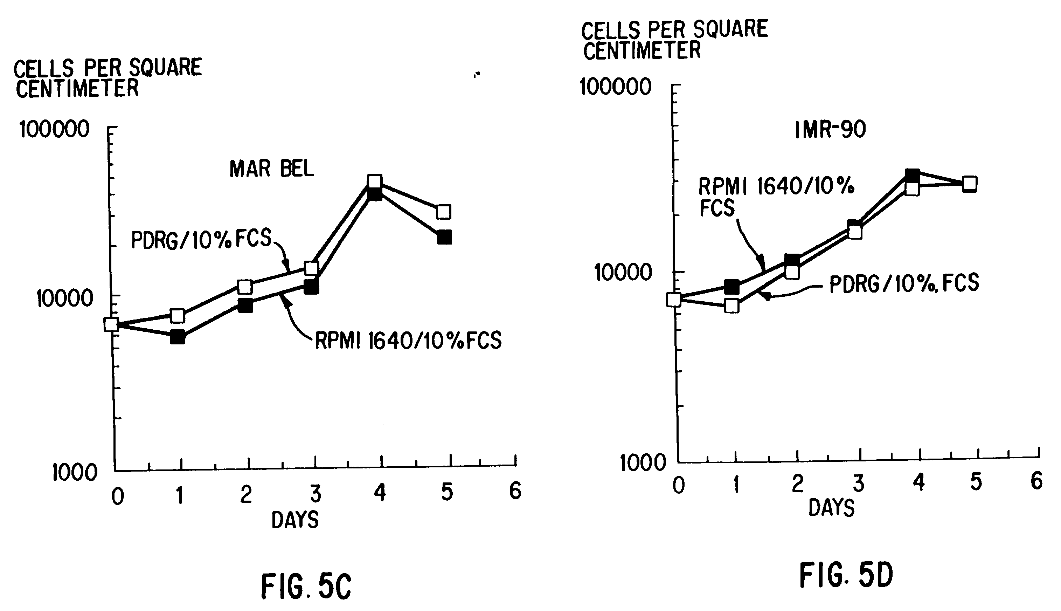

Growth curves for selected human tumor cell lines in PDRG basal growth medium and L-15 medium in atmospheric CO₂ and in RPMI 1640 medium in 5% CO₂ are presented in Figure 3. Calculation of the doubling time for each cell line in the respective medium (Table 3) indicates that the doubling times for the HOP 62 lung adenocarcinoma, the SN12K1 renal carcinoma and the XF 498 glioblastoma are slightly longer in PDRG basal growth medium in atmospheric CO₂ than in RPMI 1640 medium, under 5% CO₂. The greatest discrepancy, a 2.7-fold increase, was found for the OVCAR 5 ovarian carcinoma. In contrast, the doubling times for the M19Mel melanoma and the HT 29 colon adenocarcinoma are shorter in atmospheric CO₂ than in 5% CO₂. A growth curve for the P388 and L1210 murine lymphocytic leukemias in 5% CO₂ and in PDRG basal growth medium in atmospheric CO₂ is presented in Figure 4. Growth curves of 4 human and 1 murine fibroblast (Figure 5) in 5% CO₂ and in PDRG basal growth medium in atmospheric CO₂ are almost identical.

### Promotion of Growth of Human Tumor Cell Lines in Atmospheric CO₂ by Oxaloacetic Acid

Oxaloacetic acid, a tricarboxylic acid cycle intermediate, was evaluated for growth stimulation of each of the tumor cell lines (Figure 6). Incorporation of oxaloacetic acid into PDRG basal growth medium produced a maximum increase in cell proliferation in the HOP 62 lung adenocarcinoma of 25%, the HT 29 colon adenocarcinoma (13%), the Sn12K1 renal carcinoma (28%) and the M19Mel Melanoma (38%). Growth stimulation by oxaloacetic acid occurred over a concentration range of 0.5 to 6.0 mM for these cell lines. The XF498 glioblastoma, the OVCAR 5 ovarian carcinoma, and the K562 myelogenous leukemia were refractory to growth promotion by oxaloacetic acid.

### Optimization of PDRG Basal Growth Medium for Propagation of Leukemia Cells in Atmospheric CO₂

Adaption of tumor cell lines representing central nervous system, colon, renal, lung, melanoma and ovarian histologies to growth in atmospheric CO₂ in PDRG basal growth medium was generally complete following 2-3 passages (Figure 2). This contrasted with results obtained from the K562 chronic myelogenous leukemia. Removal of K562 from RPMI 1640 and growth in PDRG basal growth medium in atmospheric CO₂ was accompanied by a progressive decline in growth for three passages followed by 5-6 passages of generally stable growth (Figure 2). However, although growth of K562 in PDRG basal growth medium was superior to that in L-15 or RPMI 1630 (Figure 3), it was substantially less than that in RPMI 1640 and 5% CO₂ (Figure 3). These observations suggested the possibility that metabolic production of CO₂ for utilization in purine and pyrimidine biosynthesis might be limiting and prompted a detailed investigation into the effect of their supplementation on the growth of K562. Supplementation of PDRG basal growth medium with purine and pyrimidine bases or their ribo/deoxyribonucleotides resulted in minimal growth stimulation (data not shown). However, supplementation of PDRG basal growth medium with the purine nucleosides adenosine, guanosine and inosine, the pyrimidine nucleosides, cytidine and uridine, and the pyrimidine precursor, orotic acid, (Table 2) resulted in both the elimination of the lengthy period of adaption of K562 to growth in atmospheric CO₂ and considerable stimulation of growth of this cell line in atmospheric CO₂ (Figure 7). Growth of K562 in this modified PDRG basal growth medium in atmospheric CO₂ and in RPMI 1640 and 5% CO₂ were comparable. Examination of the doubling times (Td) of K562 in both media indicates similar doubling times for days 1-3 post seeding. However, for days 4-8, cells grown in modified PDRG basal growth medium had substantially shorter Tds than cells grown in RPMI 1640 (Table 4). A similar pattern emerged when comparing specific growth rates for K562 cells grown in both media (Table 4). In addition, the saturation density for K562 cells grown in the modified PDRG basal growth medium was 50% greater than that obtained with RPMI 1640 (Figure 7).

### IC₅₀ Values for Selected Drugs in Atmospheric CO₂

IC₅₀ values for adriamycin, BCNU and tamoxifen in atmospheric CO₂ and 5% CO₂ are presented in Table 5. Similar values were obtained under both experimental conditions. However, in general, cells were more sensitive to these drugs when the MTT tetrazolium assay was employed to evaluate cell viability as compared to the SRB protein assay. This enhanced sensitivity is reflected by lower IC₅₀ values and occurred both in atmospheric CO₂ and in 5% CO₂.

In short, the data presented herein clearly indicate that RPMI 1630, which utilizes dibasic sodium phosphate as a primary buffer (Moore et al, 1966, JNCI, 36:405-421) was unsatisfactory (Figure 2). L-15, developed by Leibovitz for propagation of cells in atmospheric CO₂ (Leibovitz, 1963, Amer. J. Hyg., 78:172-180) was superior to RPMI 1630 in the current study (Figure 2), but found to be unsatisfactory for utilization in metabolic assays which measure cellular viability with tetrazolium salts.

The results obtained with PDRG basal growth medium indicate that murine and a broad range of human tumor cell lines representing a number of different histologies as well as several human and murine fibroblasts can be successfully adapted to growth in atmospheric CO₂ in PDRG basal growth medium (Table 2). This medium does not require exogenous CO₂ since bicarbonate ion is eliminated as the primary buffering component.

The requirement of CO₂ for a variety of cellular biochemical processes, including purine, pyrimidine and fatty acid biosynthesis, resulted in incorporation into the PDRG basal growth medium of several components designed to facilitate metabolic production and utilization of CO₂: (1) biotin, a cofactor for carboxylation reactions, (2) pyruvic acid, a monocarboxylic acid which acts both as a metabolic source of CO₂ via decarboxylation as well as by providing a source of acetyl coenzyme A for metabolic stimulation of CO₂ production by the tricarboxylic acid cycle and (3) L-asparagine, a C₄ amino acid which can be converted intracellulary to L-aspartic acid and oxaloacetic acid by the cytosolic enzyme L-asparagine amidohydrolase (E.C. 3.5.1.1.) and the mitochondrial enzyme L-aspartate 2-oxoglutarate aminotransferase (E.C. 2.6.1.1.), respectively. In addition, oxaloacetic acid, a tricarboxylic acid cycle intermediate, was tested as a partial replacement for CO₂. Oxaloacetic acid was found, in the present study, to increase cellular proliferation in several cell lines (Figure 6) but was not included in the PDRG Basal Medium formulation because it produced marked inhibition of cell growth in the OVCAR 5 ovarian carcinoma, the XF 498 glioblastoma, and the K562 myelogenous leukemia. Additionally, oxaloacetic acid undergoes spontaneous decarboxylation resulting in alkalinization of growth medium. These results suggest that oxaloacetic acid should be evaluated for growth promotion in individual cell lines and, in instances where promotion of growth is observed, be incorporated into experimental protocols utilizing those cell lines. In addition, incorporation of purine nucleosides (adenosine, guanosine and inosine), pyrimidine nucleosides (cytidine and uridine) and the pyrimidine precursor orotic acid eliminated the period of adaption of the K562 chronic myelogenous leukemia to growth in atmospheric CO₂ and resulted in nearly identical growth in RPMI 1640 and 5% CO₂ and modified PDRG basal growth medium and atmospheric CO₂ (Figure 7, Table 4). The reduced growth of K562 in PDRG basal growth medium (Figures 2, 3, 7) and the ability of purine and pyrimidine nucleosides to eliminate this growth differential suggests that metabolic production of CO₂ for incorporation into nucleic acid precursors may be limiting in this cell line.

PDRG basal growth medium exhibits several desirable characteristics for use in a high-flux drug screening mode. First, the medium has good pH stability and buffering capacity in atmospheric CO₂. This prevents changes in medium pH during periods when cells are being seeded into assay plates as well as during drug treatment. Second, the medium exhibits the capability for large scale growth of a broad range of human tumor cell lines. Third, since D-glucose is the principal carbohydrate in the formulation, the medium is compatible for use with either tetrazolium or protein based assays for determination of cellular viability. Finally, IC₅₀ values for adriamycin, BCNU and tamoxifen were similar in cell lines maintained in PDRG basal growth medium in atmospheric CO₂ and in RPMI 1640 and a 5% CO₂ environment.

Of course, as is well known in the art, the cell culture medium of the present invention can be used in conjunction with hollow fiber and associated techniques for mass production of cells and products thereof, such as growth factors, hormones, antibodies, biological response modifiers and the like where the use of CO₂ to control the medium pH is found cumbersome, expensive, impractical or undesirable. The growth medium of the present invention lends itself to both general and specialized cell culture applications.

**Table 1.**

| Cell Lines Utilized for Growth in Atmospheric CO₂ | |
|---|---|
| Cell Line | Histology |
| XF 498 | Human Central Nervous System (Glioblastoma) |
| M19MEL | Human Melanoma |
| OVCAR 5 | Human Ovarian Adenocarcinoma |
| A 2780 | Human Ovarian Adenocarcinoma |
| HOP 62 | Human Lung Adenocarcinoma |
| SN12K1 | Human Renal Cell Carcinoma |
| HT 29 | Human Colon Adenocarcinoma |
| K 562 | Human Chronic Myelogenous Leukemia |
| P 388 | Murine Lymphocytic Leukemia |
| L 1210 | Murine Lymphocytic Leukemia |
| | |
| MRC-5 | Human Fibroblast |
| CCD-19 LU | Human Fibroblast |
| Mar Bel | Human Fibroblast |
| IMR-90 | Human Fibroblast |
| 3T3 | Murine Fibroblast |

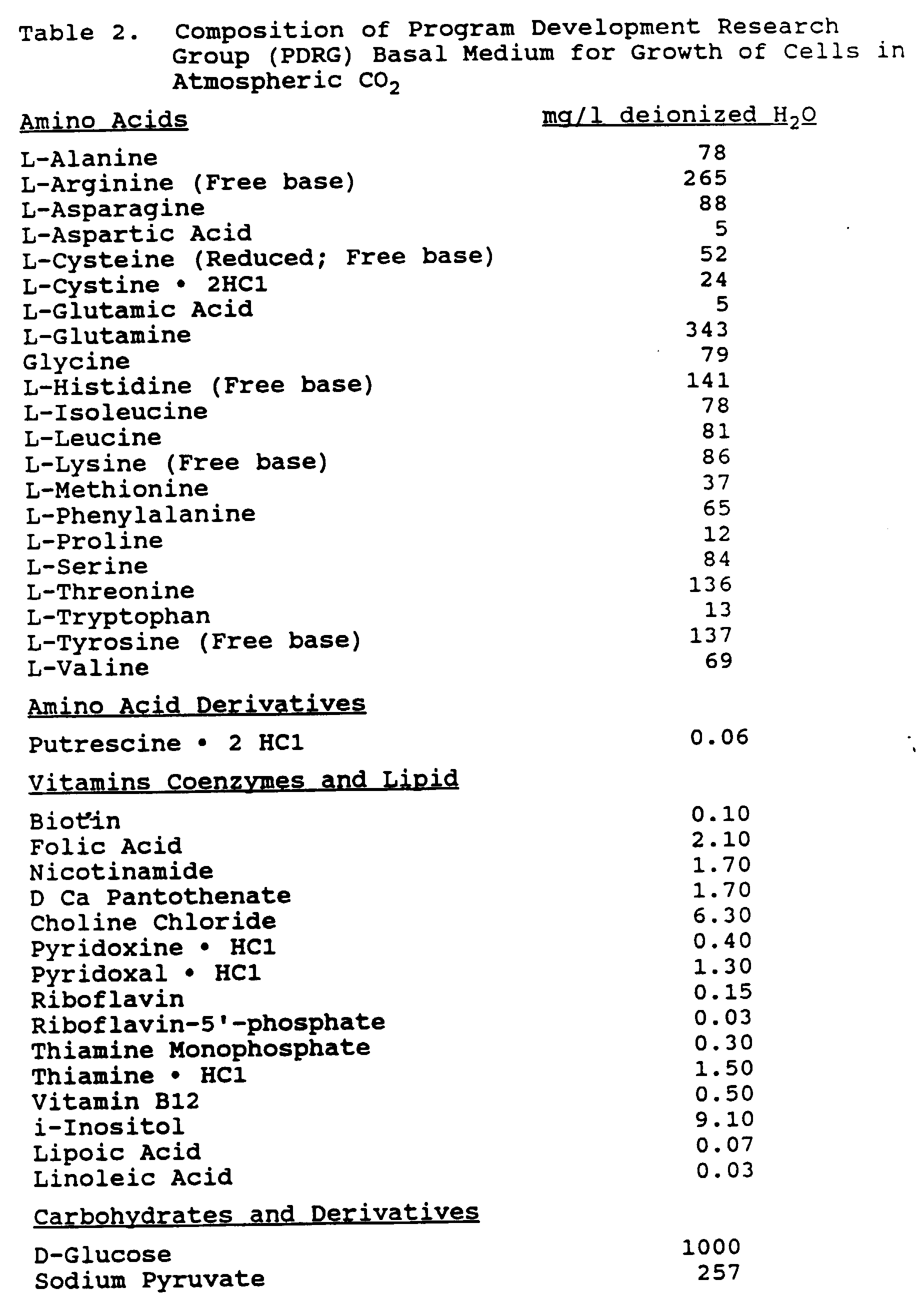

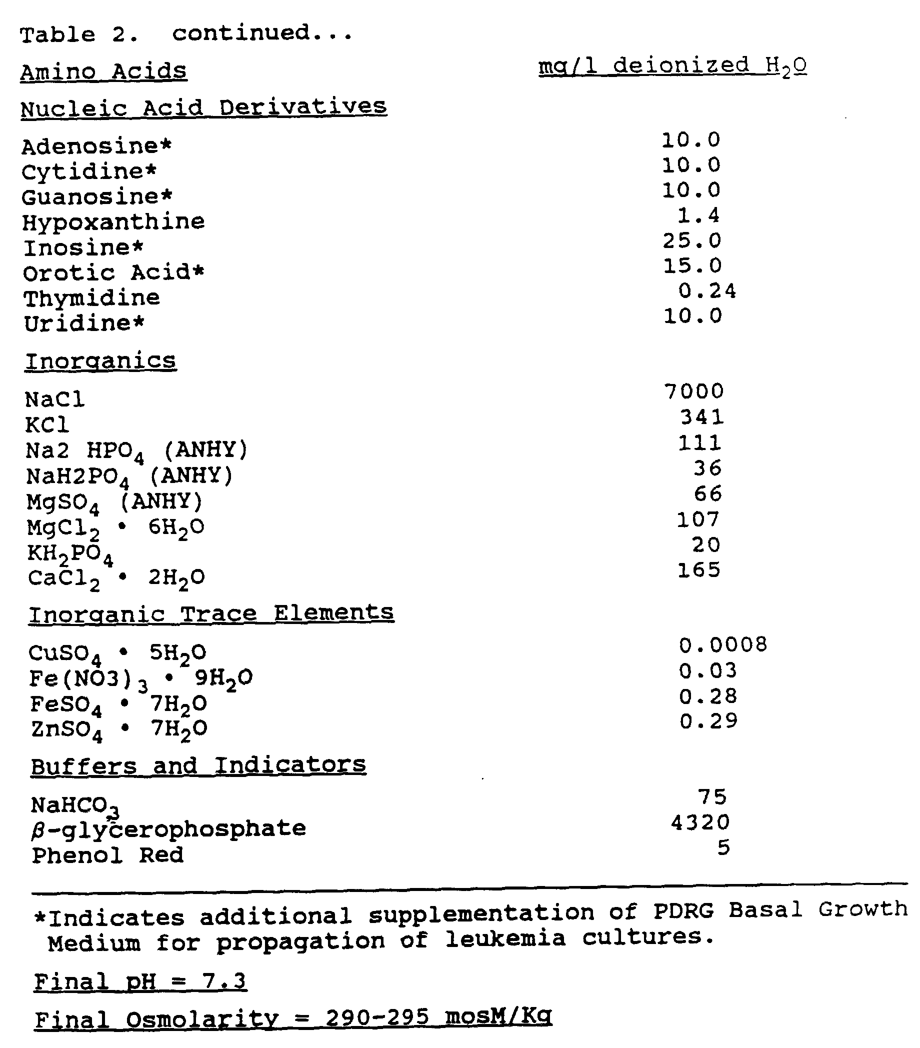

**Table 3.**

| Doubling times (Td) of cell lines of RPMI 1640 and PDRG basal growth media | | |
|---|---|---|
| | Td (hours) | |
| Cell Line | RPMI 1640 +5% CO₂ | PDRG Basal Growth Medium +0.04% CO₂ |
| HOP 62 | 28.5 | 41.0 |
| SN12K1 | 17.8 | 25.0 |
| M19MEL | 53.6 | 22.4 |
| HT 29 | 31.7 | 28.7 |
| XF 498 | 41.0 | 50.6 |
| OVCAR 5 | 24.0 | 65.0 |
| K562 | 16.5 | 83.3 |
| Doubling times (Td) were calculated from the day 2 cell counts presented in Figure 3 and 5 (for K562) according to the method of Skehan (29), as outlined in the section on Materials and Methods. | | |

**Table 4.**

| Doubling times (Td) and specific growth rates (SGR) of K562 myelogenous leukemia cells in RPMI 1640 medium, PDRG basal growth medium and PDRG basal growth medium supplemented with nucleic acid precursors.* | | | | | | |
|---|---|---|---|---|---|---|
| Day | RPMI 1640 + 5% CO₂ | | PDRG Basal Growth Medium + 0.04% CO₂ | | PDRG Basal Growth Medium + 0.04% CO₂ + Nucleic Acid Precursors* | |
| | Td (hours) | SGR | Td (hours) | SGR | Td (hours) | SGR |
| 1 | 19 | (139) | 28 | (80) | 20 | (129) |
| 2 | 17 | (174) | 83 | (22) | 22 | (115) |
| 3 | 26 | ( 92) | 59 | (32) | 28 | ( 82) |
| 4 | 63 | ( 30) | 44 | (46) | 37 | ( 57) |
| 5 | -- | -- | -- | -- | -- | -- |
| 6 | 491 | (3.5) | 85 | (22) | 63 | ( 30) |
| 7 | 576 | (2.9) | 66 | (29) | 108 | ( 17) |
| 8 | | ( 0) | 78 | (24) | 164 | ( 11) |

| | | | | | | |
|---|---|---|---|---|---|---|
| *Additional supplementation of PDRG Basal Growth Medium with adenosine, cytidine, guanosine, uridine, inosine and orotic acid as indicated in Table 2. | | | | | | |

Doubling times (Td) and specific growth rates (SGR, percent increase in cell number per day) were calculated from growth curve data (Figure 5), according to the method of Skehan (29).

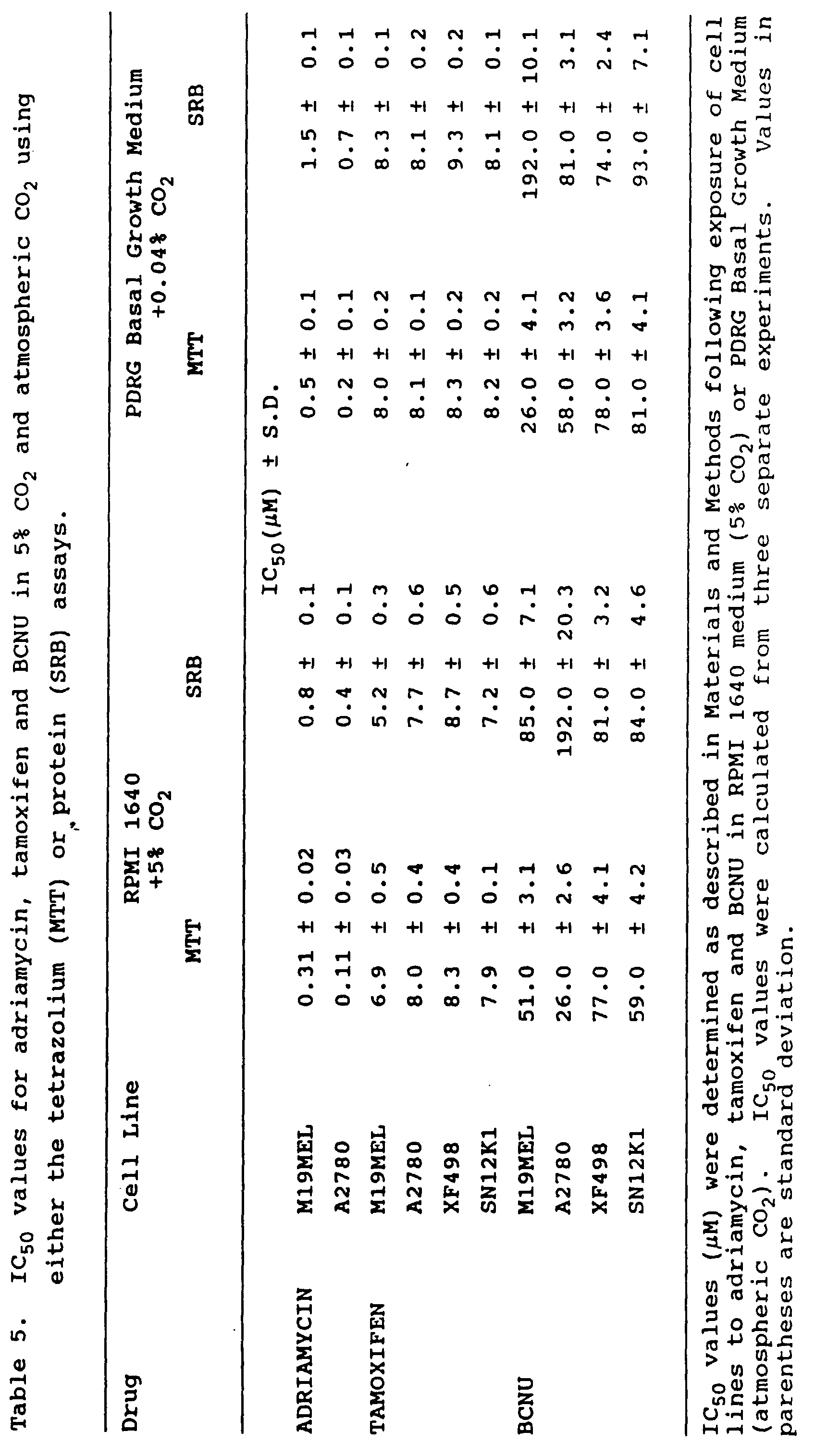

## Claims

1. A growth medium for the maintenance, growth or propagation of cells in a CO₂-independent atmosphere, composed of the following ingredients: 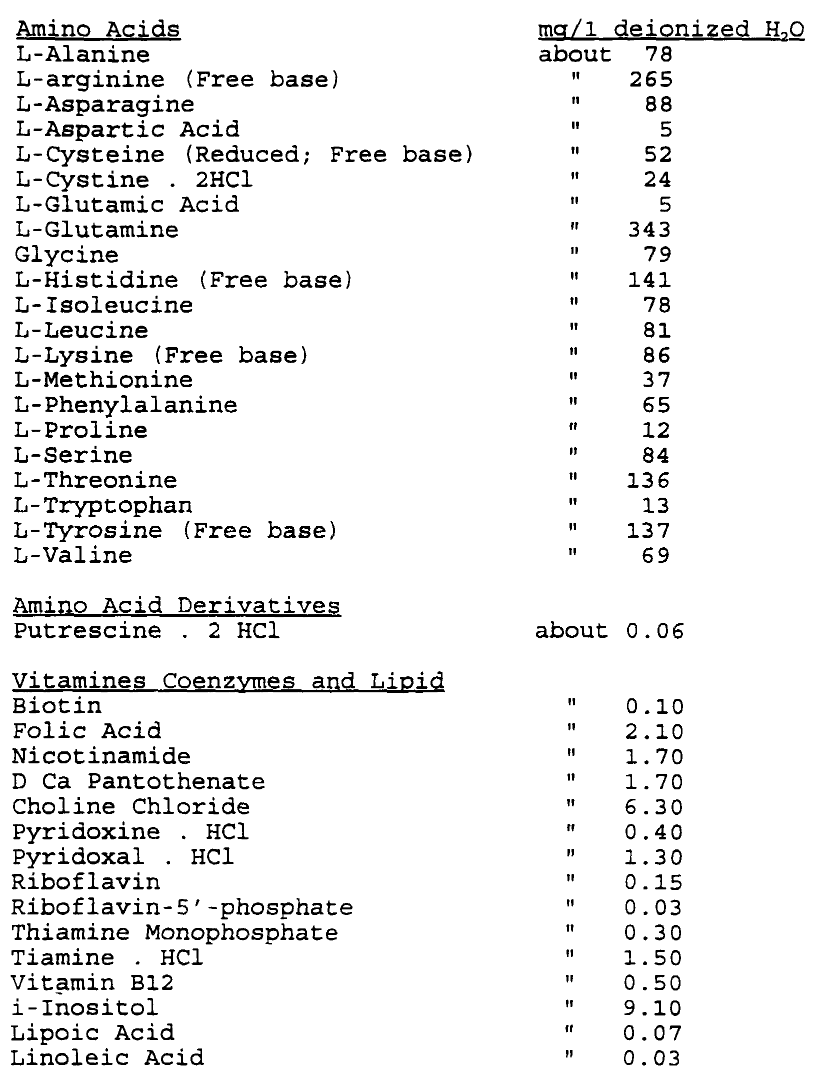 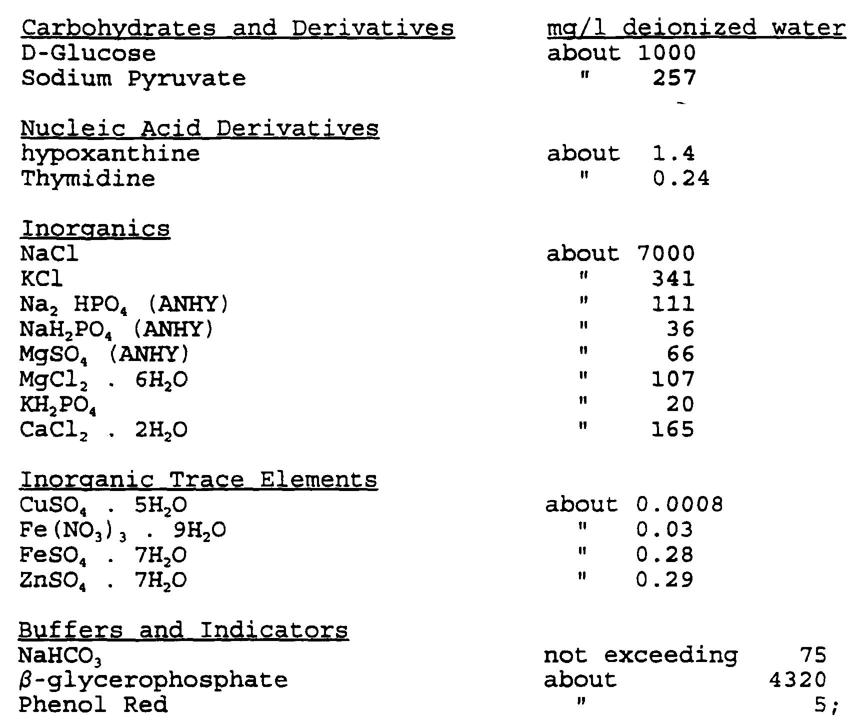 and having a final pH of about 7.3 and final osmolarity of about 290-295 mos/kg.

2. A growth medium as claimed in claim 1, for the propagation of leukaemia cultures, additionally supplemented with a nucleic acid derivative selected from the group consisting of:
adenosine (about 10.0 mg/l deionized H₂O), cytidine (about 10.0 mg/l deionized H₂O), guanosine (about 10.0 mg/l deionized H₂O), inosine (about 25.0 mg/l deionized H₂O), orotic acid (about 15.0 mg/l deionized H₂O) and uridine (about 10.0 mg/l deionized H₂O).

3. A growth medium as claimed in claims 1 or 2, further comprising oxaloacetic acid at a concentration of 0.5-6.0 mM.

4. A method for culturing cells, comprising growing said cells in a growth medium as claimed in any one of claims 1-3 in a CO₂-independent atmosphere.

5. A method as claimed in claim 4, further comprising the use of a hollow-fiber technique.

6. A method for in vitro screening of anticancer agents, comprisng growing tumor cells in vitro in a growth medium as claimed in any one of claims 1-3 in the absence and presence of an anticancer agent, wherein a decrease in growth of the cells in the presence of the anticancer agent, is indicative of the anticancer potency of said agent.

## Patentansprüche

1. Wachstumsmedium zur Erhaltung, zum Züchten oder zur Fortpflanzung von Zellen in einer CO₂-unabhängigen Atmosphäre, die aus folgenden Zutaten zusammengesetzt ist: 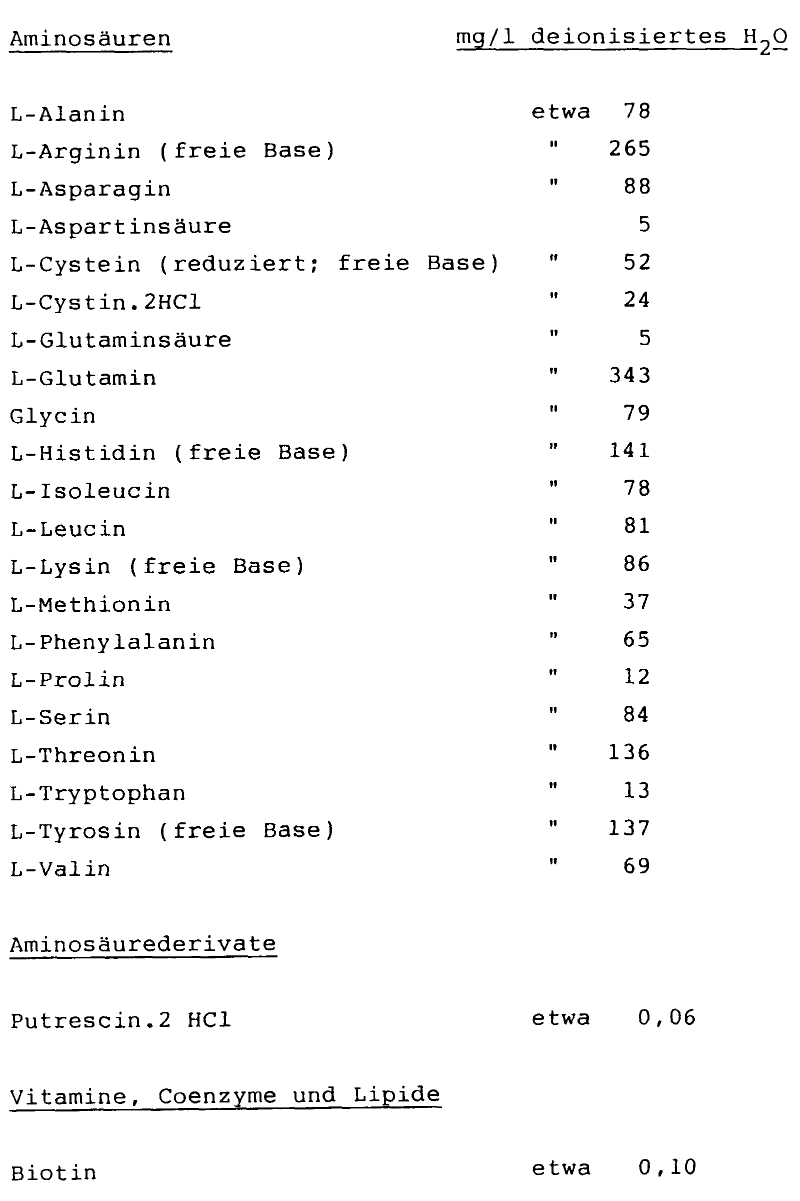 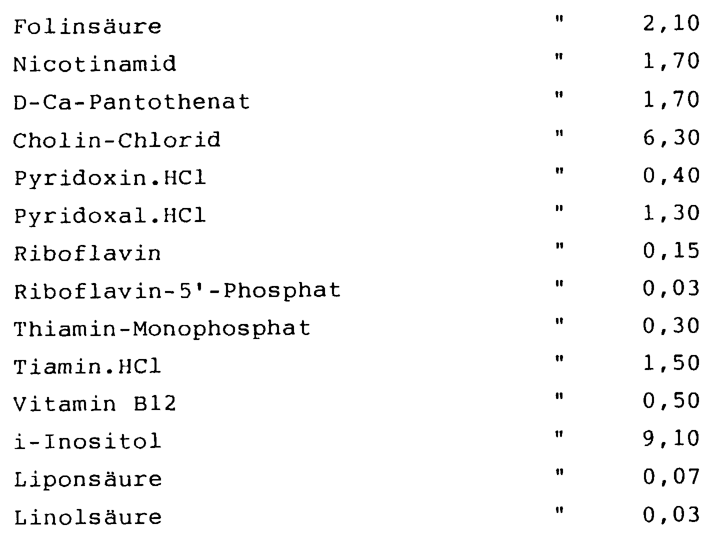 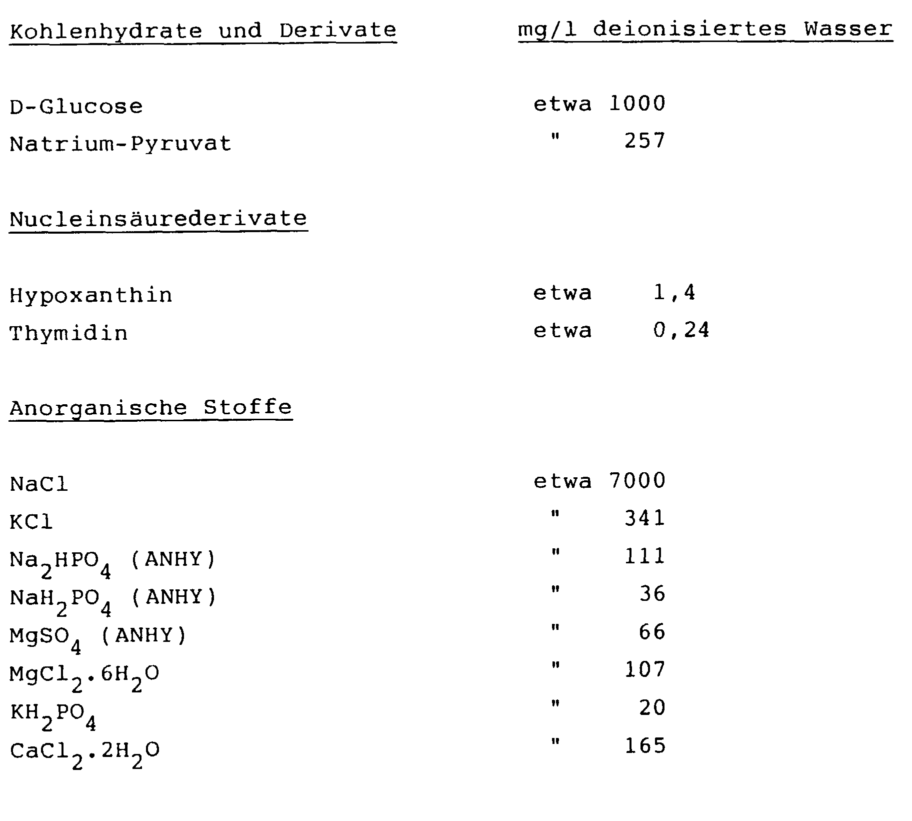 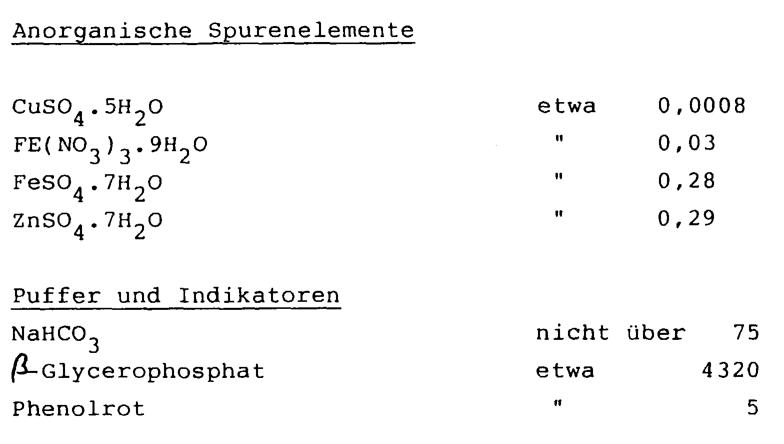 und mit einem pH-Endwert von etwa 7,3 und einer End-Osmolarität von etwa 290-295 mos/kg.

2. Wachstumsmedium nach Anspruch 1 zur Vermehrung von Leukämiekulturen, das zusätzlich mit einem Nukleinsäurederivat ergänzt ist, das aus der folgenden Gruppe ausgewählt ist:
Adenosin (etwa 10,0 mg/l deionisiertes H₂O), Cytidin (etwa 10,0 mg/l deionisiertes H₂O), Guanosin (etwa 10,0 mg/l deionisiertes H₂O), Inosin (etwa 25,0 mg/l deionisierts H₂O), Orotsäure (etwa 15,0 mg/l deionisiertes H₂O) und Uridin (etwa 10,0 mg/l deionisiertes H₂O).

3. Wachstumsmedium nach Anspruch 1 oder 2, das außerdem Oxalessigsäure mit einer Konzentration von 0,5 bis 6,0 mM enthält.

4. Verfahren zum Züchten von Zellen, welches das Züchten der Zellen in einem Wachstumsmedium nach einem der Ansprüche 1 bis 3 in einer CO₂-unabhängigen Atmosphäre umfaßt.

5. Verfahren nach Anspruch 4, das außerdem die Anwendung einer Hohlfasertechnik umfaßt.

6. Verfahren zum in-vitro-Testen von Anti-Krebs-Agenzien, welches das Züchten von Tumorzellen in vitro in einem Wachstumsmedium nach einem der Ansprüche 1 bis 3 in Abwesenheit und Anwesenheit eines Anti-Krebs-Agens umfaßt, wobei eine Wachstumsabnahme der Zellen in Anwesenheit des Anti-Krebs-Agens die Anti-Krebs-Potenz des Agens anzeigt.

## Revendications

1. Milieu de croissance permettant l'entretien, la croissance ou la propagation de cellules dans une atmosphère indépendamment de sa teneur en CO₂, composée des ingrédients suivants : 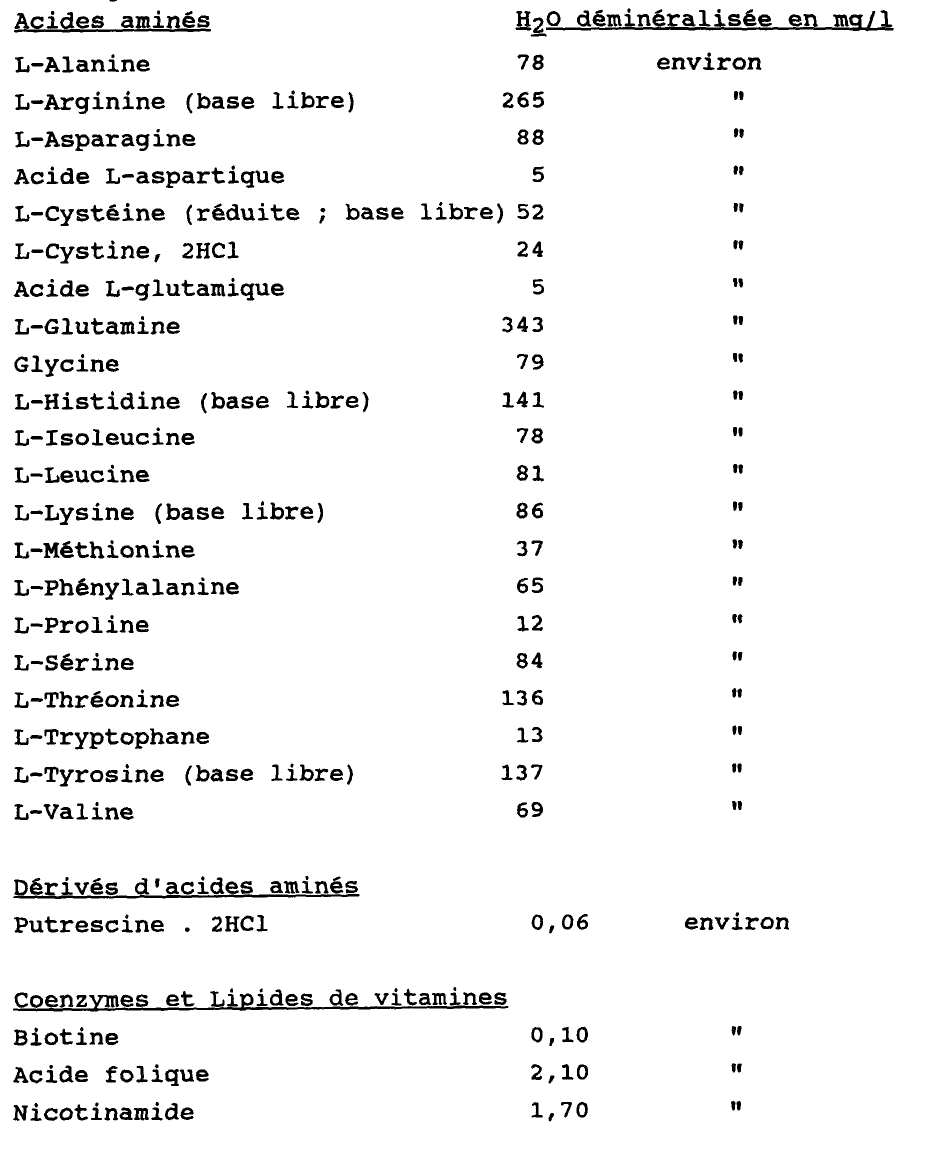 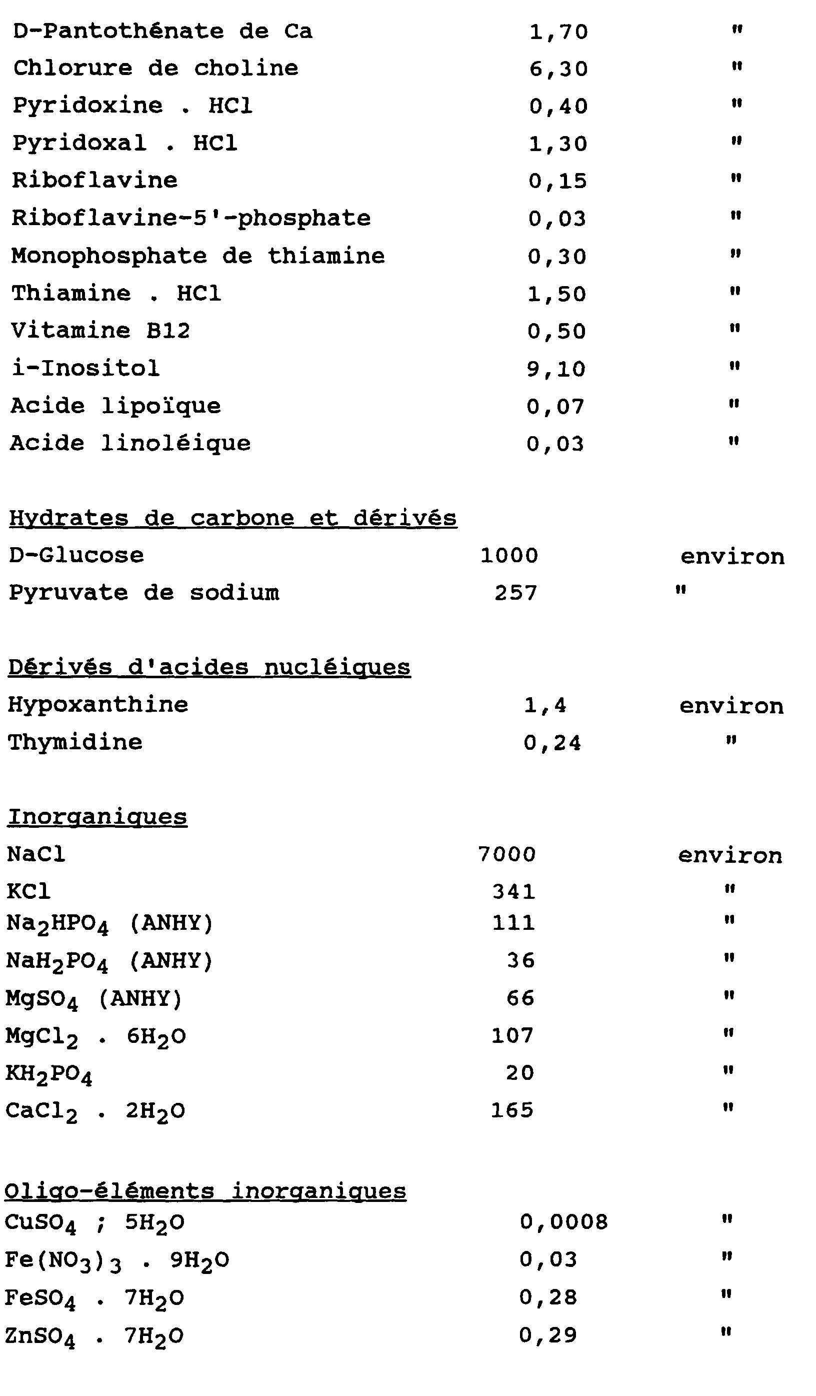 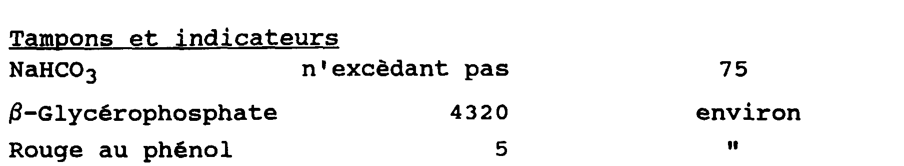 et ayant un pH final de 7,3 environ et une osmolarité finale de 290-295 mos/kg environ.

2. Milieu de croissance selon la revendication 1, permettant la propagation de cultures de cellules de leucémie, complété, de plus, par un dérivé d'acide nucléique choisi dans le groupe consistant en :
adénosine (environ 10,0 mg/l d'H₂O déminéralisée), cytidine (environ 10,0 mg/l d'H₂O déminéralisée), guanosine (environ 10,0 mg/l d'H₂O dminéralisée), inosine (environ 25,0 mg/l d'H₂O déminéralisée), acide orotique (environ 15,0 mg/l d'H₂O déminéralisée), et uridine (environ 10,0 mg/l d'H₂O déminéralisée).

3. Milieu de croissance selon la revendication 1 ou 2, comprenant en outre de l'acide oxaloacétique à une concentration de 0,5 - 6,0 mM.

4. Procédé de culture de cellules, comprenant l'étape consistant à faire croître lesdites cellules dans un milieu de croissance selon l'une quelconque des revendication 1 à 3, dans une atmosphère indépendamment de la teneur en CO₂ de cette dernière.

5. Procédé selon la revendication 4, comprenant en outre l'utilisation de la technique de la fibre creuse.

6. Procédé permettant le criblage in vitro d'agents anticancéreux, comprenant la croissance de cellules tumorales in vitro dans un milieu de croissance selon l'une quelconque des revendications 1 à 3, en l'absence et en présence d'un agent anticancéreux, procédé dans lequel une réduction de la croissance des cellules en présence de l'agent anticancéreux indique le pouvoir anticancéreux dudit agent.
